# EUROPEAN PATENT APPLICATION

(11) **EP 4 609 804 A1**
(43) Date of publication of application: **03.09.2025**
(21) Application number: 23896425.8
(22) Date of filing: 31.10.2023
(51) Int. Cl.: A61B 17/12, A61B 17/00

(54) **LEFT ATRIAL APPENDAGE OCCLUDER**

(30) Priority: 30.11.2022 CN 202211521167
(71) Applicant: Guangdong Pulse Medical Technology Co., Ltd, Zhuhai, Guangdong 519080 (CN)
(72) Inventor: RUAN, Chengmin, Zhuhai, Guangdong 519080 (CN); JIANG, Yalun, Zhuhai, Guangdong 519080 (CN); SUN, Yanhong, Zhuhai, Guangdong 519080 (CN)
(74) Representative: Kransell & Wennborg KB
(86) International application number: PCT/CN2023/128837
(87) International publication number: WO 2024/114271

(57) **Abstract**

The present application provides a left atrial appendage occluder, comprising: a fixed disc, wherein the fixed disc is provided with a first connecting structure, and the first connecting structure is provided with a first threaded section; and an occlusion disc, wherein the occlusion disc is provided with a second connecting structure, and the second connecting structure is provided with a second threaded section. The first threaded section and the second threaded section are each provided with a threaded structure and are in threaded engagement. The occlusion disc is further provided with a conveying thread configured for threaded fit with a conveying steel cable. The rotation direction when screwing the threaded structure is opposite to the rotation direction when screwing the conveying thread. At least one of the first connecting structure and the second connecting structure further comprises a moving section. After the first threaded section and the second threaded section are screwed to a preset distance, the first threaded section is disengaged from the second threaded section, and the second threaded section or the first threaded section is in fit with the moving section, allowing the fixed disc and the occlusion disc to move by a predetermined distance in the axial direction of the moving section. The present application solves the problem in the prior art that a left atrial appendage occluder is prone to loosening when in connection and fit with the conveying steel cable.

## Description

This application claims priority to Chinese Patent Application No. 202211521167.3, filed with the Chinese Patent Office on November 30, 2022, entitled "Left Atrial Appendage Occluder", the entirety of which is incorporated herein by reference.

### Technical Field

The present application relates to the technical field of medical devices, and in particular to a left atrial appendage occluder.

### Background Art

All cardiovascular left atrial appendage occluders currently available on the market are mostly cut stents in the form of single-piece inner plugs formed by laser cutting and subsequent heat treatment of metal pipes, or left atrial appendage occluder stents formed by using metal wires that are woven and subsequently heat-treated, or welded with different types of cut stents in the middle, wherein the middle connecting parts thereof are all connected by means of laser welding, which is an immovable connection mode, and implants are connected to delivery systems by threads. All the left atrial appendage occluders currently available on the market have occlusion discs and fixation discs connected by means of laser welding, which are all designed as undetachable and unadjustable structures.

Common structures of left atrial appendage occluders include plug type and double disc type. A left atrial appendage occluder having a double disc type structure consists of a sealing device and a fixing device and usually has the advantage of allowing repeated withdrawal and release, but has the following defects.
(1) All the left atrial appendage occluders currently available on the market are divided into single-piece inner plug structures and structures with inner plugs and outer covers. The single-piece inner plug structure has the defect that it cannot effectively occlude the ostium of the left atrial appendage during clinical practice, leading to occurrence of residual leakage, which tends to form a residual cavity that is not easily endothelialized.
(2) The left atrial appendage occluder structure with an inner plug and an outer cover has the defect that the outer cover of the left atrial appendage occluder is not completely suitable in size after implantation during clinical practice due to the irregularity of the left atrial appendage. If the occlusion disc is too large, it will cause abrasion of the pulmonary veins and mitral valve. If the occlusion disc is too small, a residual cavity will be formed, which is not easily endothelialized.
(3) All the left atrial appendage occluder products are structurally inseparable or cannot be freely assembled, and thus it is not possible to select an appropriate left atrial appendage occluder product for a patient according to the actual clinical needs.
(4) The left atrial appendage occluder products have a uniform height, which has requirements for the depth of the left atrial appendage.

In order to solve the defects existing in the left atrial appendage occluders mentioned above, there is a technology relating to an improved left atrial appendage occluder, including an anchoring part, a connecting part, and a covering part, wherein the connecting part consists of a threaded connector, a hollow limiter, and a clamping element. Although the improved structure enables detachability of the anchoring part and the covering part, it brings new problems and has obvious defects. Namely, during connection in a surgical procedure for implanting the left atrial appendage occluder into a heart, the occluder is connected to a delivery steel cable by means of threaded connection, the threads are tightened when rotated in a forward direction, and the nut is loosened when rotated in a reverse direction. This design is not only complex in structure, but also results in a serious risk of loosening of the nuts of the occlusion disc and the fixation disc, due to loosening of the middle threaded connection with the rotation of the delivery steel cable when the product is released and freed. Since the heart is in a diastolic state, it is extremely difficult to locate and re-screw the two discs through imaging once they are loosened. Moreover, a spherical shape of the middle connecting part can adjust the angle of the occlusion disc. When encountering an irregular left atrial appendage structure, the cover-type occlusion disc will cause incomplete occlusion on one side, resulting in a risk of residual leakage, that is, incomplete occlusion, which tends to produce a residual thrombus cavity.

### Summary

A main object of the present application is to provide a left atrial appendage occluder to solve the problem in the prior art that a left atrial appendage occluder is prone to loosening when connected and fitted with a delivery steel cable.

In order to achieve the above-mentioned object, a left atrial appendage occluder is provided according to an optional implementation of the present application, which comprises: a fixation disc, wherein the fixation disc has a first connecting structure, and the first connecting structure has a first threaded section; and an occlusion disc, wherein the occlusion disc has a second connecting structure, the second connecting structure has a second threaded section, the first threaded section and the second threaded section each have a threaded structure and are threadedly engaged with each other, the occlusion disc further has a delivery thread for being threadedly fitted with a delivery steel cable, a rotation direction in which the threaded structure is screwed is opposite to a rotation direction in which the delivery thread is screwed, at least one of the first connecting structure and the second connecting structure further comprises a moving section, and after the first threaded section and the second threaded section are threadedly screwed to a preset distance, the first threaded section is disengaged from the second threaded section, and the second threaded section or the first threaded section is in fit with the moving section, allowing the fixation disc and the occlusion disc to move by a predetermined distance in an axial direction of the moving section.

In an optional embodiment, the direction of rotation of the threaded structure is left-handed.

In an optional embodiment, the first connecting structure has a first moving section as the moving section, the first moving section is connected to the first threaded section, and the first moving section is farther away from the occlusion disc than the first threaded section.

In an optional embodiment, the second connecting structure has a second moving section as the moving section, the second moving section is connected to the second threaded section, and the second moving section is farther away from the fixation disc than the second threaded section.

In an optional embodiment, the occlusion disc further comprises an occlusion disc body, the second connecting structure comprises a large-diameter section and a small-diameter section axially connected in sequence, the large-diameter section is connected to the occlusion disc body, the small-diameter section comprises the second threaded section and the second moving section, and the second moving section is located between the second threaded section and the large-diameter section.

In an optional embodiment, the fixation disc further comprises a fixation disc body, the first connecting structure has a sleeve structure, and an inner wall surface of the sleeve structure comprises the first threaded section and the first moving section.

In an optional embodiment, the first connecting structure has a first moving section as the moving section, the first threaded section is internally threaded, and an inner diameter of the first moving section is greater than or equal to the maximum diameter of the threaded structure of the first threaded section.

In an optional embodiment, the second connecting structure has a second moving section as the moving section, the second threaded section is externally threaded, and an outer diameter of the second moving section is less than or equal to the minimum diameter of the threaded structure of the second threaded section.

In an optional embodiment, the predetermined distance is 2 mm to 6 mm.

In an optional embodiment, the left atrial appendage occluder further comprises an anti-rotation structure, the anti-rotation structure comprises an anti-rotation protrusion and an anti-rotation groove, one of the anti-rotation protrusion and the anti-rotation groove is provided on the first connecting structure, the other thereof is provided on the second connecting structure, the anti-rotation protrusion is located on the moving section, at least a part of the anti-rotation groove is located at the threaded structure, and the anti-rotation protrusion extends into the anti-rotation groove and impedes relative rotation of the fixation disc and the occlusion disc after the first threaded section is disengaged from the second threaded section.

In an optional embodiment, the first connecting structure has a sleeve structure, the first threaded section is located at one of two ends of the sleeve structure that is close to the occlusion disc, the anti-rotation groove extends from the first threaded section in a direction away from the occlusion disc and communicates with an end surface of the sleeve structure, the second connecting structure has the second threaded section and a second moving section serving as the moving section, the second threaded section is externally threaded, and the anti-rotation protrusion is located at the second moving section.

In an optional embodiment, there is at least one anti-rotation protrusion, and when there are a plurality of anti-rotation protrusions, the individual anti-rotation protrusions are arranged at intervals in a circumferential direction of the moving section.

In an optional embodiment, the occlusion disc is of a trapezoidal structure in an unstretched state, and slanted sides of the trapezoidal structure are inclined in a direction close to an axis of the occlusion disc, along a direction close to the fixation disc.

In an optional embodiment, the trapezoidal structure has a height of 2 mm to 6 mm, and the trapezoidal structure has a base angle of 70° to 85°.

By applying the technical solution of the present application, the direction of rotation of the threaded structures on the first threaded section and the second threaded section is set to be opposite to the direction of rotation of the delivery thread. In this way, when the delivery steel cable is used to be fitted with the occlusion disc, even if the delivery steel cable and the occlusion disc are rotated, only the threaded fitting relationship between the delivery steel cable and the occlusion disc can be changed due to the opposite direction of rotation of the threaded structures. Even if the occlusion disc is driven to rotate, the connection relationship between the occlusion disc and the fixation disc cannot be changed, so that the first threaded section and the second threaded section between the fixation disc and the occlusion disc will not move in a direction of disengagement and separation from each other, but will have a further screwed effect, thereby ensuring non-loosening of the fixation disc and the occlusion disc so as to ensure the stability and safety of the left atrial appendage occluder during surgery. Meanwhile, this embodiment is further provided with a moving section. The moving section can provide an adjustable space for the axial distance between the fixation disc and the occlusion disc after they are connected together. Specifically, when they are being connected, the first threaded section and the second threaded section are threadedly connected together, and then as the tightening operation is continued, the first threaded section and the second threaded section which are brought into contact at one end will be disengaged at the other end. The disengagement at this time is merely the disattachment of the threaded structures, which will not affect the connection relationship between the fixation disc and the occlusion disc. After the first threaded section is disengaged from the second threaded section, the axial positions of the first connecting structure and the second connecting structure relative to each other are no longer constrained by the threaded structures, and they can move along the moving section, thereby adjusting the distance between the fixation disc and the occlusion disc, so that the left atrial appendage occluder can be automatically adjusted according to the size of the left atrial appendage, is applicable to the sizes of left atrial appendages with different depths, and thus has wider applicability. In addition, since the fixation disc and the occlusion disc of this embodiment are fitted by a threaded detachable connection, fixation discs and occlusion discs of different specifications can be freely matched, so that fixation discs and occlusion discs of different specifications can be adaptively selected and matched in response to situations such as complex internal structures, irregular morphologies, and inconsistent sizes of different left atrial appendages, so as to form more targeted left atrial appendage occluders.

### Brief Description of Drawings

The accompanying drawings constituting part of the present application are intended to provide a further understanding of the present application. The illustrative embodiments of the present application and descriptions thereof are intended to explain the present application and do not constitute an improper limitation of the present application. In the drawings:
FIG.1 shows a schematic structural diagram of a left atrial appendage occluder of the present application;
FIG.2 shows a schematic structural diagram of a fixation disc in FIG.1;
FIG.3 shows a schematic structural diagram of an occlusion disc in FIG.1;
FIG.4 shows an enlarged view of portion P in FIG.3;
FIG.5 shows a schematic structural diagram of a first connecting structure;
FIG.6 shows a side view of FIG.5;
FIG.7 shows a schematic structural diagram of a second connecting structure;
FIG.8 shows a side view of FIG.7;
FIG.9 shows a schematic structural diagram of the first connecting structure fitted with the second connecting structure; and
FIG. 10 is a schematic structural diagram of FIG. 9 from another viewing angle.

Here, the above drawings include the following reference numerals:
10. fixation disc; 11. first connecting structure; 111. first threaded section; 112. first moving section; 12. fixation disc body; 20. occlusion disc; 21. second connecting structure; 211. second threaded section; 212. second moving section; 213. large-diameter section; 214. small-diameter section; 22. occlusion disc body; 30. anti-rotation structure; 31. anti-rotation protrusion; 32. anti-rotation groove.

### Detailed Description of Embodiments

It should be noted that embodiments in the present application and the features in the embodiments can be combined with each other in the absence of conflict. The present application will be described in detail below with reference to the drawings and in combination with the embodiments.

In order to solve the problem in the prior art that a left atrial appendage occluder is prone to loosening when connected and fitted with a delivery steel cable, the present application provides a left atrial appendage occluder.

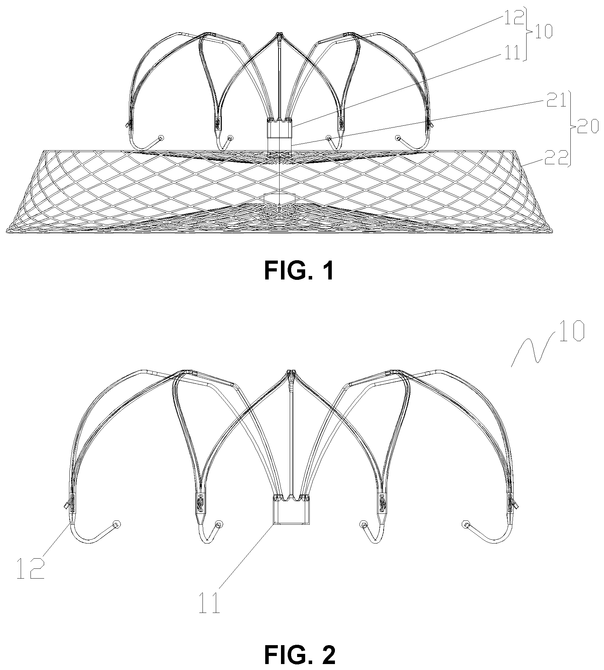

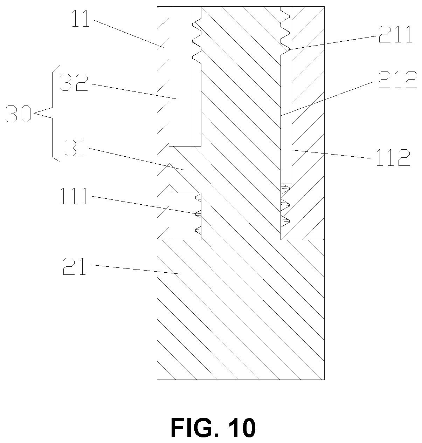

A left atrial appendage occluder as shown in FIGS. 1 to 10 includes a fixation disc 10 and an occlusion disc 20. The fixation disc 10 has a first connecting structure 11, and the first connecting structure 11 has a first threaded section 111. The occlusion disc 20 has a second connecting structure 21, and the second connecting structure 21 has a second threaded section 211. The first threaded section 111 and the second threaded section 211 each have a threaded structure and are threadedly engaged with each other. The occlusion disc 20 further has a delivery thread for being threadedly fitted with a delivery steel cable. A rotation direction in which the threaded structure is screwed is opposite to a rotation direction in which the delivery thread is screwed. At least one of the first connecting structure 11 and the second connecting structure 21 further includes a moving section. After the first threaded section 111 and the second threaded section 211 are threadedly screwed to a preset distance, the first threaded section 111 is disengaged from the second threaded section 211, and the second threaded section 211 or the first threaded section 111 is in fit with the moving section, allowing the fixation disc 10 and the occlusion disc 20 to move by a predetermined distance in an axial direction of the moving section.

In this embodiment, the direction of rotation of the threaded structures on the first threaded section 111 and the second threaded section 211 is set to be opposite to the direction of rotation of the delivery thread. In this way, when the delivery steel cable is used to be fitted with the occlusion disc 20, even if the delivery steel cable and the occlusion disc 20 are rotated, only the threaded fitting relationship between the delivery steel cable and the occlusion disc 20 can be changed due to the opposite direction of rotation of the threaded structures. Even if the occlusion disc 20 is driven to rotate, the connection relationship between the occlusion disc 20 and the fixation disc 10 cannot be changed, so that the first threaded section 111 and the second threaded section 211 between the fixation disc 10 and the occlusion disc 20 will not move in a direction of disengagement and separation from each other, but will have a further screwed effect, thereby ensuring non-loosening of the fixation disc 10 and the occlusion disc 20 so as to ensure the stability and safety of the left atrial appendage occluder during surgery. Meanwhile, this embodiment is further provided with a moving section. The moving section can provide an adjustable space for the axial distance between the fixation disc 10 and the occlusion disc 20 after they are connected together. Specifically, when they are being connected, the first threaded section 111 and the second threaded section 211 are threadedly connected together, and then as the tightening operation is continued, the first threaded section 111 and the second threaded section 211 which are brought into contact at one end will be disengaged at the other end. The disengagement at this time is merely the disattachment of the threaded structures, which will not affect the connection relationship between the fixation disc 10 and the occlusion disc 20. After the first threaded section 111 is disengaged from the second threaded section 211, the axial positions of the first connecting structure 11 and the second connecting structure 21 relative to each other are no longer constrained by the threaded structures, and they can move along the moving section, thereby adjusting the distance between the fixation disc 10 and the occlusion disc 20, so that the left atrial appendage occluder can be automatically adjusted according to the size of the left atrial appendage, is applicable to the sizes of left atrial appendages with different depths, and thus has wider applicability. In addition, since the fixation disc 10 and the occlusion disc 20 of this embodiment are fitted by a threaded detachable connection, fixation discs 10 and occlusion discs 20 of different specifications can be freely matched, so that fixation discs 10 and occlusion discs 20 of different specifications can be adaptively selected and matched in response to situations such as complex internal structures, irregular morphologies, and inconsistent sizes of different left atrial appendages, so as to form more targeted left atrial appendage occluders.

Preferably, since the delivery steel cable is generally tightened by being rotated rightward and loosened by being rotated leftward, the direction of rotation of the delivery thread is right-handed, and thus the direction of rotation of the threaded structures is left-handed. This not only conforms to the general usage habits, but also can ensure an anti-loosening effect. Of course, when the delivery thread is left-handed, the direction of rotation of the threaded structures can be correspondingly adjusted to be right-handed.

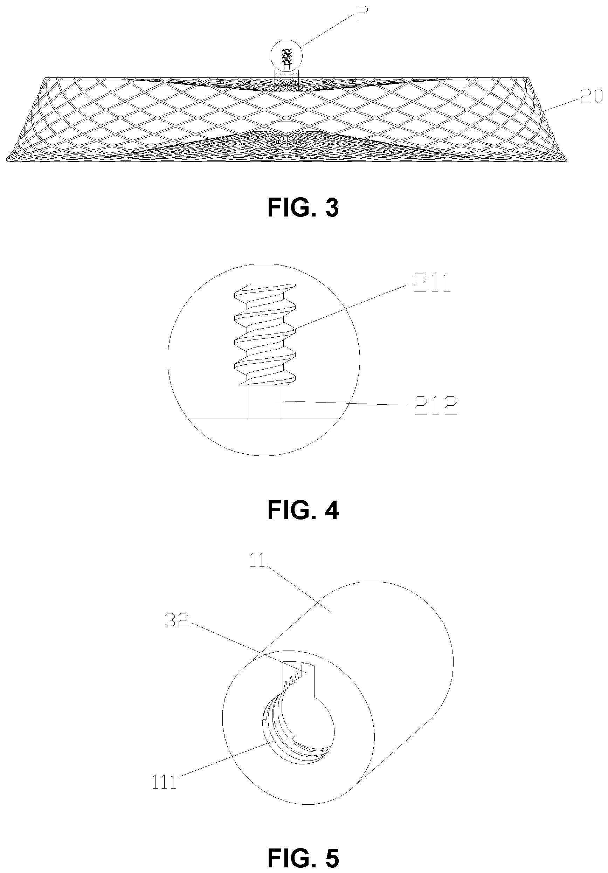

As shown in FIG. 5 to 10, in this embodiment, the first connecting structure 11 and the second connecting structure 21 are each provided with a moving section. Specifically, the first connecting structure 11 has a first moving section 112 as the moving section, and the second connecting structure 21 has a second moving section 212 as the moving section, wherein the first moving section 112 is connected to the first threaded section 111, and the first moving section 112 is farther away from the occlusion disc 20 than the first threaded section 111. The second moving section 212 is connected to the second threaded section 211, and the second moving section 212 is farther away from the fixation disc 10 than the second threaded section 211. In this way, when the first threaded section 111 and the second threaded section 211 are tightened to a certain degree and disengaged from each other, the first moving section 112 is fitted with the second threaded section 211 such that the second threaded section 211 is movable axially at the first moving section 112, and the second moving section 212 is aligned and fitted with the first threaded section 111 such that the first threaded section 111 is movable axially at the second moving section 212, thereby enabling an axial movable adjustment of the first connecting structure 11 and the second connecting structure 21 after they are connected together, thus enabling a positional adjustment between the fixation disc 10 and the occlusion disc 20.

In addition to the provision of the moving section on each of the first connecting structure 11 and the second connecting structure 21, the moving section may also be provided on only one of the first connecting structure 11 and the second connecting structure 21. Taking, as an example, a case where the second connecting structure 21 is provided with a moving section, namely, the second moving section 212 is provided and fitted as described above while there is no structure at the first moving section 112 on the first connecting structure 11, the second threaded section 211 may pass through the first connecting structure 11 and has no fitting relationship with the first connecting structure 11, among others.

As shown in FIGS. 3, 4, 7, and 8, the occlusion disc 20 of this embodiment further includes an occlusion disc body 22. The occlusion disc body 22 is a mesh structure, which has filaments converging at their ends. The second connecting structure 21 includes a large-diameter section 213 and a small-diameter section 214 axially connected in sequence, wherein one end of the large-diameter section 213 is connected to an end of the occlusion disc body 22 at which the individual filaments converge, and the other end of the large-diameter section 213 is connected to the small-diameter section 214, while the small-diameter section 214 includes the second threaded section 211 and the second moving section 212, and the second moving section 212 is located between the second threaded section 211 and the large-diameter section 213. In other words, the large-diameter section 213, the second moving section 212, and the second threaded section 211 are provided in this order along the axial direction of the second connecting structure 21. In this way, the second connecting structure 21 can be connected with both the occlusion disc body 22 and the fixation disc 10. Meanwhile, the positional relationship between the second moving section 212 and the second threaded section 211 can ensure fitting of the first threaded section 111 with the moving section when the first threaded section 111 and the second threaded section 211 are tightened to be disengaged from each other, thereby ensuring an adjustable effect.

As shown in FIGS. 2, 5, and 6, the fixation disc 10 of this embodiment further includes a fixation disc body 12. The fixation disc body 12 has a plurality of hook-shaped structures provided circumferentially. One end of each of the plurality of hook-shaped structures converges toward the middle. The first connecting structure 11 has a sleeve structure. One end of the sleeve structure is connected to the end at which the hook-shaped structures converge. A hollow part of the sleeve structure is the space allowing the second connecting structure 21 to extend thereinto. An inner wall surface of the sleeve structure includes the first threaded section 111 and the first moving section 112, and the first threaded section 111 is located at an end of the sleeve structure that is remote from the hook-shaped structure, that is to say, the first threaded section 111 is located at one of the two ends of the sleeve structure that is close to the occlusion disc 2. The first threaded section 111 does not cover the entire length of the sleeve structure, thus there is a part of the inner wall surface without a threaded structure. A portion, where the part of the inner wall surface without a threaded structure is located, is the first moving section 112. In this way, during mounting and connection, the second threaded section 211 may be aligned with the hollow part of the sleeve structure, and then extended into the hollow part and threadably fitted with the first threaded section 111. As the two sections are continuously tightened, a distance by which the second connecting structure 21 extends into the first connecting structure 11 is continuously lengthened. When the first threaded section 111 is disengaged from the second threaded section 211, the first threaded section 111 is located on the outer side of the second moving section 212, and the second threaded section 211 is located on the inner side of the first moving section 112, thereby enabling axial movement and adjustment.

In this embodiment, the first threaded section 111 is internally threaded, and the second threaded section 211 is externally threaded. In order to avoid interference between the first moving section 112 and the second threaded section 211, in this embodiment, the inner diameter of the first moving section 112 is greater than or equal to the maximum diameter of the threaded structure of the first threaded section 111. The maximum diameter of the threaded structure of the first threaded section 111 is equivalent to the maximum diameter of the threaded structure of the second threaded section 211, thus the inner diameter of the first moving section 112 is greater than or equal to the maximum diameter of the threaded structure of the second threaded section 211. In this way, the second threaded section 211 can move freely and smoothly in the axial direction when it is located in the first moving section 112. Similarly, in order to avoid interference between the second moving section 212 and the first threaded section 111, in this embodiment, the outer diameter of the second moving section 212 is less than or equal to the minimum diameter of the threaded structure of the second threaded section 211. The minimum diameter of the threaded structure of the second threaded section 211 is equivalent to the minimum diameter of the threaded structure of the first threaded section 111, thus the outer diameter of the second moving section 212 is less than or equal to the minimum diameter of the threaded structure of the first threaded section 111. In this way, the first threaded section 111 can move freely and smoothly in the axial direction when it is located in the second moving section 212.

Preferably, in this embodiment, the predetermined axially adjustable distance between the fixation disc 10 and the occlusion disc 20 is set to 2 mm to 6 mm. Such adjustment range is more conformable to the environment of the left atrial appendage.

It should be noted that the specific structure and configuration of each of the first connecting structure 11 and the second connecting structure 21 mentioned above are not limited to those listed above in this embodiment and can be adjusted as needed. For example, the structural forms of the first connecting structure 11 and the second connecting structure 21 mentioned above can be interchanged.

In this embodiment, in order to ensure movement of the fixation disc 10 and the occlusion disc 20 only in the axial direction during adjustment and avoid their circumferential rotations, the left atrial appendage occluder of this embodiment further includes an anti-rotation structure 30, wherein the anti-rotation structure 30 includes an anti-rotation protrusion 31 and an anti-rotation groove 32, one of the anti-rotation protrusion 31 and the anti-rotation groove 32 is provided on the first connecting structure 11, and the other thereof is provided on the second connecting structure 21. After the first threaded section 111 is disengaged from the second threaded section 211, the anti-rotation protrusion 31 extends into the anti-rotation groove 32 and impedes the relative rotation of the fixation disc 10 and the occlusion disc 20, thereby achieving the effect of preventing rotation of the fixation disc 10 and the occlusion disc 20 relative to each other by fitting of the anti-rotation protrusion 31 into the anti-rotation groove 32.

Specifically, since the axially adjustable part mainly lies in the moving section, in this embodiment, the anti-rotation protrusion 31 is provided on the moving section, and at least a part of the anti-rotation groove 32 is provided at the threaded structure. The part which is provided on the first connecting structure 11 and the part which is provided on the second connecting structure 21 can be specifically adjusted according to actual needs. This embodiment is described by taking the anti-rotation protrusion 31 provided on the second moving section 212 and the anti-rotation groove 32 provided in the first threaded section 111 as an example. Of course, it is also possible to provide the anti-rotation protrusion 31 on the first moving section 112 and provide the anti-rotation groove 32 in the second threaded section 211, or the above two configurations may be employed at the same time.

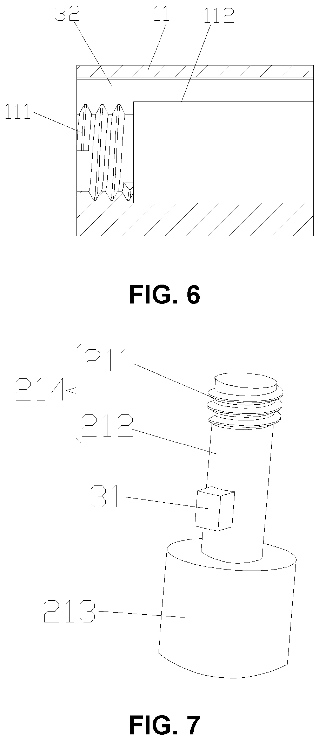

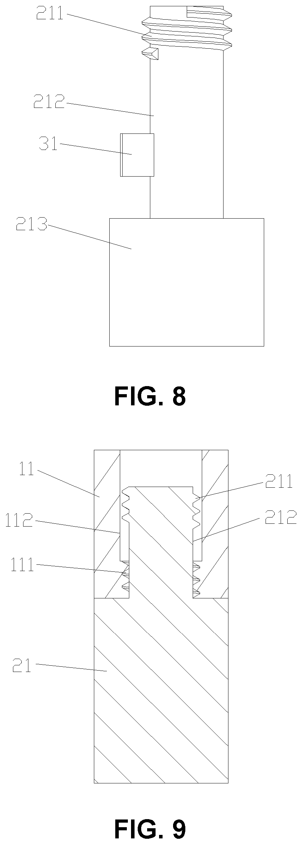

Specifically, the anti-rotation groove 32 is located at one end of the sleeve structure that is close to the occlusion disc 20. In other words, the anti-rotation groove 32 and the first threaded section 111 are located at the same end of the sleeve structure, as shown in FIGS. 7 and 8. In this way, the inner wall surface of the sleeve structure can have a threaded structure as the first threaded section 111, and the anti-rotation groove 32 is provided at a position of the sleeve structure radially corresponding to the first threaded section 111. Moreover, in order to ensure that the anti-rotation protrusion 31 can extend into the anti-rotation groove 32 during axial movement, the anti-rotation groove 32 communicates with the end surface of the sleeve structure. Meanwhile, the anti-rotation groove 32 may extend by a distance in a direction away from the occlusion disc 20, or even pass through both ends of the sleeve structure, so as to avoid interference between the sleeve structure and the anti-rotation protrusion 31. Correspondingly, the anti-rotation protrusion 31 is located on the circumferential side surface of the second moving section 212 and protrudes radially. The anti-rotation protrusion 31 is adapted to the shape of the anti-rotation groove 32, thereby achieving an anti-rotation effect.

Considering that the anti-rotation protrusion 31 and the anti-rotation groove 32 may not be aligned after the first threaded section 111 and the second threaded section 211 are rotated out of engagement, a certain distance is left between the anti-rotation protrusion 31 and the second threaded section 211 in this embodiment. Since the predetermined axially adjustable distance between the fixation disc 10 and the occlusion disc 20 is 2 to 6 mm, the axial spacing between the anti-rotation protrusion 31 and the second threaded section 211 may be correspondingly 2 to 6 mm. Therefore, after the first threaded section 111 is disengaged from the second threaded section 211, the occlusion disc 20 can be continuously rotated in a small range to adjust the relative positions of the anti-rotation protrusion 31 and the anti-rotation groove 32. When the anti-rotation protrusion 31 and the anti-rotation groove 32 are aligned with each other, the anti-rotation protrusion 31 immediately extends into the anti-rotation groove 32 to achieve anti-rotation.

Optionally, the number of the anti-rotation protrusions 31 and the anti-rotation grooves 32 may be set as needed, and the individual anti-rotation protrusions 31 or anti-rotation grooves 32 may be arranged at intervals in the circumferential direction of the moving section. In this embodiment, only one anti-rotation protrusion 31 and one anti-rotation groove 32 are provided. Of course, it is also possible to provide two or more anti-rotation protrusions 31 and two anti-rotation grooves 32. Taking provision of two anti-rotation protrusions and two anti-rotation grooves as an example, the two anti-rotation protrusions 31 are located on the circumferentially opposite sides of the second moving section 212, respectively, and correspondingly, the two anti-rotation grooves 32 are located in the opposite sides of the sleeve structure. In other words, the two anti-rotation grooves 32 pass through the opposite sides of the sleeve along the radial direction of the sleeve structure, which can improve the anti-rotation effect.

As shown in FIGS. 1 and 3, in this embodiment, the occlusion disc 20 is of a trapezoidal structure in an unstretched state. More precisely, the cross section of the occlusion disc 20 through the center line is of a trapezoidal structure, and the slanted sides of the trapezoidal structure are inclined in the direction close to the axis of the occlusion disc 20, along the direction close to the fixation disc 10. In this way, the use of a trapezoidal design can effectively avoid the problems occurring during implantation of an outer cover type occluder, such as residual leakage caused by an excessively small occlusion disc, which will form a residual cavity that is not easily endothelialized, and abrasion of the pulmonary veins and mitral valve caused by an excessively large occlusion disc. Even if the size of the occlusion disc can just match the shape of the left atrial appendage, the occlusion disc has a thin edge, which will continuously abrade the surrounding tissues during the continuous contraction and relaxation cycle of the heart. Therefore, the occlusion disc 20 with a trapezoidal design has higher deformation flexibility and can better fit the anatomical structures of the left atrial appendages with different shapes, which significantly improves the occluding effect and greatly reduces the abrasion of the surrounding tissues such as the pulmonary veins and mitral valve.

During surgery, the internal traction of the fixation disc 10 enables the occlusion disc 20 to become an elliptical spindle structure. As the one-stop ablation and occlusion surgery is being carried out, the elastic mesh tube of the occlusion disc 20 with a trapezoidal design can fit more closely to the ostium of the left atrial appendage as the ablation progresses. The outer disc of the occlusion disc 20 fits smoothly with the ostium of the left atrial appendage without protruding from the ostium of the left atrial appendage, thereby having no effect on both the left superior pulmonary vein and the mitral valve, with no formation of a new residual cavity or residual leakage.

Preferably, the trapezoidal structure has a height of 2 mm to 6 mm, the trapezoidal structure has a base angle of 70° to 85°, and the height and the base angle are in a matching proportional relationship, so that the shape of the occlusion disc 20 can fit more tightly against the inner wall of the left atrial appendage.

It should be noted that the term "a plurality of" in the above embodiments refers to at least two.

It can be seen, from the above description, that the above embodiments of the present application achieve the following technical effects.
1. The problem in the prior art is solved, in which a left atrial appendage occluder is prone to loosening when connected and fitted with a delivery steel cable.
2. The fitting of the delivery steel cable and the occlusion disc to each other will not change the connection relationship between the occlusion disc and the fixation disc, thereby ensuring non-loosening of the fixation disc and the occlusion disc so as to ensure the stability and safety of the left atrial appendage occluder during surgery.
3. The distance between the fixation disc and the occlusion disc can be adjusted, so that the left atrial appendage occluder can be automatically adjusted according to the size of the left atrial appendage, is applicable to the sizes of left atrial appendages with different depths, and thus has wider applicability.
4. Fixation discs and occlusion discs of different specifications can be freely matched, so that fixation discs and occlusion discs of different specifications can be adaptively selected and matched in response to situations such as complex internal structures, irregular morphologies, and inconsistent sizes of different left atrial appendages, so as to form more targeted left atrial appendage occluders.
5. The anti-rotation structure impedes the relative rotation of the fixation disc and the occlusion disc to ensure the axial adjustment effect.
6. The use of a trapezoidal design allows the outer disc of the occlusion disc to fit smoothly with the ostium of the left atrial appendage without protruding from the ostium of the left atrial appendage, thereby having no effect on both the left superior pulmonary vein and mitral valve, with no formation of a new residual cavity or residual leakage.

Obviously, the embodiments described above are only some embodiments, rather than all embodiments, of the present application. All the other embodiments obtained by those of ordinary skill in the art based on the embodiments in the present application without inventive efforts should fall within the scope of protection of the present application.

The above description is merely illustrative of the preferred embodiments of the present application and is not intended to limit the present application. It will be appreciated by those skilled in the art that the present application may have various modifications and variations. Any modifications, equivalent replacements, improvements, and the like made within the spirit and principle of the present application shall be included within the scope of protection of the present application.

## Claims

1. A left atrial appendage occluder, **characterized by** comprising:
a fixation disc (10), wherein the fixation disc (10) has a first connecting structure (11), and the first connecting structure (11) has a first threaded section (111); and
an occlusion disc (20), wherein the occlusion disc (20) has a second connecting structure (21), the second connecting structure (21) has a second threaded section (211), the first threaded section (111) and the second threaded section (211) each have a threaded structure and are threadedly engaged with each other, the occlusion disc (20) further has a delivery thread for being threadedly fitted with a delivery steel cable, a rotation direction in which the threaded structure is screwed is opposite to a rotation direction in which the delivery thread is screwed, at least one of the first connecting structure (11) and the second connecting structure (21) further comprises a moving section, and after the first threaded section (111) and the second threaded section (211) are threadedly screwed to a preset distance, the first threaded section (111) is disengaged from the second threaded section (211), and the second threaded section (211) or the first threaded section (111) is in fit with the moving section, allowing the fixation disc (10) and the occlusion disc (20) to move by a predetermined distance in an axial direction of the moving section.

2. The left atrial appendage occluder according to claim 1, wherein the rotation direction of the threaded structure is left-handed.

3. The left atrial appendage occluder according to claim 1 or claim 2, wherein
the first connecting structure (11) has a first moving section (112) as the moving section, the first moving section (112) is connected to the first threaded section (111), and the first moving section (112) is farther away from the occlusion disc (20) than the first threaded section (111).

4. The left atrial appendage occluder according to any one of claims 1-3, wherein
the second connecting structure (21) has a second moving section (212) as the moving section, the second moving section (212) is connected to the second threaded section (211), and the second moving section (212) is farther away from the fixation disc (10) than the second threaded section (211).

5. The left atrial appendage occluder according to claim 4, wherein the occlusion disc (20) further comprises an occlusion disc body (22), the second connecting structure (21) comprises a large-diameter section (213) and a small-diameter section (214) axially connected in sequence, the large-diameter section (213) is connected to the occlusion disc body (22), the small-diameter section (214) comprises the second threaded section (211) and the second moving section (212), and the second moving section (212) is located between the second threaded section (211) and the large-diameter section (213).

6. The left atrial appendage occluder according to claim 3, wherein the fixation disc (10) further comprises a fixation disc body (12), the first connecting structure (11) has a sleeve structure, and an inner wall surface of the sleeve structure comprises the first threaded section (111) and the first moving section (112).

7. The left atrial appendage occluder according to any one of claims 1-6, wherein
the first connecting structure (11) has a first moving section (112) as the moving section, the first threaded section (111) is internally threaded, and an inner diameter of the first moving section (112) is greater than or equal to a maximum diameter of a threaded structure of the first threaded section (111).

8. The left atrial appendage occluder according to any one of claims 1-7, wherein
the second connecting structure (21) has a second moving section (212) as the moving section, the second threaded section (211) is externally threaded, and an outer diameter of the second moving section (212) is less than or equal to a minimum diameter of a threaded structure of the second threaded section (211).

9. The left atrial appendage occluder according to any one of claims 1-8, wherein the predetermined distance is 2 mm to 6 mm.

10. The left atrial appendage occluder according to any one of claims 1-9, wherein the left atrial appendage occluder further comprises an anti-rotation structure (30), the anti-rotation structure (30) comprises an anti-rotation protrusion (31) and an anti-rotation groove (32), one of the anti-rotation protrusion (31) and the anti-rotation groove (32) is provided on the first connecting structure (11), the other thereof is provided on the second connecting structure (21), the anti-rotation protrusion (31) is located on the moving section, at least a part of the anti-rotation groove (32) is located at the threaded structure, and the anti-rotation protrusion (31) extends into the anti-rotation groove (32) and impedes relative rotation of the fixation disc (10) and the occlusion disc (20) after the first threaded section (111) is disengaged from the second threaded section (211).

11. The left atrial appendage occluder according to claim 10, wherein the first connecting structure (11) has a sleeve structure, the first threaded section (111) is located at one of two ends of the sleeve structure that is close to the occlusion disc (20), the anti-rotation groove (32) extends from the first threaded section (111) in a direction away from the occlusion disc (20) and communicates with an end surface of the sleeve structure, the second connecting structure (21) has the second threaded section (211) and a second moving section (212) serving as the moving section, the second threaded section (211) is externally threaded, and the anti-rotation protrusion (31) is located at the second moving section (212).

12. The left atrial appendage occluder according to claim 10, wherein at least one anti-rotation protrusion (31) is provided, and when a plurality of anti-rotation protrusions (31) are provided, the individual anti-rotation protrusions (31) are arranged at intervals in a circumferential direction of the moving section.

13. The left atrial appendage occluder according to any one of claims 1-12, wherein the occlusion disc (20) is of a trapezoidal structure in an unstretched state, and slanted sides of the trapezoidal structure are inclined in a direction close to an axis of the occlusion disc (20), along a direction close to the fixation disc (10).

14. The left atrial appendage occluder according to claim 13, wherein the trapezoidal structure has a height of 2 mm to 6 mm, and the trapezoidal structure has a base angle of 70° to 85°.
